# EUROPÄISCHE PATENTSCHRIFT

(11) **EP 0 495 153 B1**
(45) Veröffentlichungstag und Bekanntmachung des Hinweises auf die Patenterteilung: **30.11.1994**
(21) Anmeldenummer: 91110165.7
(22) Anmeldetag: 20.06.1991
(51) Int. Cl.: C07C 17/38, C08J 11/02

(54) **Verfahren zum Rückgewinnen von halogeniertem Kohlenwasserstoff oder dergleichen aus Kunststoff-Schäumen oder dergleichen**
Process for recovering halogenated hydrocarbons and the like from plastic foams and the like
Procédé de récupération des hydrocarbures halogénés et similaires de mousses plastiques et similaires

(30) Priorität: 14.01.1991 DE 4100875
(43) Veröffentlichungstag der Anmeldung: 22.07.1992
(73) Patentinhaber: ERE Emmer Recycling und Entsorgung GmbH, D-90596 Schwanstetten (DE)
(72) Erfinder: Hoberg, Heinz, Prof. Dr.-Ing., W-51 Aachen Laurensberg (DE); Christiani, Joachim, Dipl.-Ing., W-6600 Saarbrücken 2 (DE); Bender, Martin, W-5100 Aachen (DE)
(74) Vertreter: Rost, Jürgen, Dipl.-Ing.

(56) Entgegenhaltungen:
- WO-A-91/02638
- DE-A- 3 933 811
- DE-U- 8 815 199
- KUNSTSTOFFE, Bd. 79, Nr. 4, April 1989, München, DE; Seiten 315-319; J. HESSELBACH et al: "Recycling von Fluorchlorkohlenwasserstoffen beim Herstellen von PUR-Weichschaum"

## Beschreibung

Die Erfindung betrifft ein Verfahren mit den im Oberbegriff des Anspruchs 1 angegebenen Merkmalen.

Kunststoff-Schäume enthalten je nach Einsatz unterschiedliche Mengen von halogeniertem Kohlenwasserstoff (im folgenden FCKW genannt). FCKW ist z.B. unter dem Warenzeichen Frigen bekannt. Je nach Hersteller sind die Frigene vom Typ R11/R12 in unterschiedlichen Mengenverhältnissen vorhanden. Den wesentlichen Anteil stellt in der Regel jedoch R11. Ein bevorzugtes Einsatzgebiet von Kunststoff-Schäumen ist die Isolation von Kühlgeräten, bei denen Polyurethanschaum (PUR) mit einem Frigen-Gesamtgehalt von etwa 15% verwendet wird.

Bei der Aufbereitung von alten Kühlschränken werden zum Zwecke der FCKW-Abreicherung die Poren der Kunststoff-Schäume durch Zerkleinern, Verpressen oder eine Kombination beider Verfahren ausgeschlossen. Es muß dabei dafür gesorgt werden, daß der FCKW aus Gründen des Umweltschutzes nicht in die Atmosphäre abgegeben, sondern rückgewonnen wird.

Bei einem bekannten Verfahren der eingangs beschriebenen Art wird der FCKW in die Prozeßluft abgegeben, die als Trägermedium wirkt (ugl. z.B. DE-U 8 815 199). Durch eine Tieftemperaturbehandlung der Prozeßluft wird dann der FCKW rückgewonnen. Wegen des niedrigen Dampfdrucks von R11 (Siedepunkt 23,8°C) ist diese Temperaturbehandlung aber sehr aufwendig, weil Temperaturen in der Größenordnung von -60°C erreicht werden müssen, um eine gute Abreicherung zu erzielen. Wesentlich früher auftretende Wasserkondensation und Eisbildung erschweren die Realisation dieses Verfahrens. Dies gilt wegen des noch niedrigeren Siedepunkts (-29,8°C) erst recht bei der Rückgewinnung von R12.

Der Erfindung liegt die Aufgabe zugrunde, diese Nachteile zu vermeiden und ein Verfahren anzugeben, bei dem die Rückgewinnung von FCKW insbesondere im Hinblick auf die anzuwendenden Temperaturen unproblematisch ist.

Die Lösung dieser Aufgabe wird durch die im Anspruch 1 angegebenen Merkmale erreicht.

Durch die Verwendung von wasserdampf als Trägermedium ergeben sich folgende Vorteile:
Wasserdampf als Trägermedium erfährt in einem engen Temperaturbereich eine Phasenumwandlung, so daß eine Abtrennung gasförmig-flüssig erleichtert wird.
FCKW ist in der Gasphase im Wasserdampf gut löslich, in der flüssigen Phase aber minimal löslich.
Wasserdampf zeigt in Näherung Idealgasverhalten, so daß das Lösungsverhalten von gasförmigem FCKW unproblematisch ist.
Durch die Abfuhr eines reinen FCKW-Gasstroms können auch Schäume entsorgt werden, die mit unterschiedlichem FCKW hergestellt wurden.
Trotz der hohen Verdampfungsenthalpie von Wasser ist die Verwendung von Wasserdampf auch energiewirtschaftlich günstig, weil die gute Energiespeicherfähigkeit von Wasserdampf gegenüber Luft den Einsatz eines minimalen Trägermediumstroms gestattet.
Die Verwendung von Wasserdampf vermeidet die Entzündung des Schaummaterials, so daß sich ein vorteilhaftes Brandverhütungsverhalten ergibt.

Vorteilhafte weitere Ausbildungen des erfindungsgemäßen Verfahrens sind Gegenstand der Ansprüche 2 bis 7.

So wird vor dem Überführen des FCKW in den Wasserdampf der Kunststoff-Schaum vorzugsweise mechanisch behandelt, insbesondere zerkleinert und/oder verpreßt.

Die Abreicherung des FCKW-Gehalts durch Wasserdampf kann unter Atmosphärendruck oder, um das Eindringen von Luft in das System zu verhindern, unter einem geringen Überdruck erfolgen.

Die Trennung des FCKW vom Trägermedium Wasserdampf erfolgt vorteilhaft in einem Kondensator, der als Kühler-Abscheider-Kombination ausgebildet ist. Zur Kondensation von Wasser wird das Gemisch also abgekühlt.

Der in diesem Kondensator anfallende gasförmige FCKW kann dann in wenigstens einer Kondensationsstufe, die aus Verdichter, Kühler und Abscheider besteht, verflüssigt und in der flüssigen Form abgefüllt und rückgewonnen werden.

Das im ersten Kondensator anfallende Wasser kann über eine Pumpe dem Dampferzeuger und von dort der Kunststoffschaum-Behandlung zugeführt werden. Wasserverluste ergeben sich daher allenfalls durch evtl. vorhandene Undichtigkeiten und im Bereich der Schleusen durch geringe Mengen mitausgetragenen Dampfes.

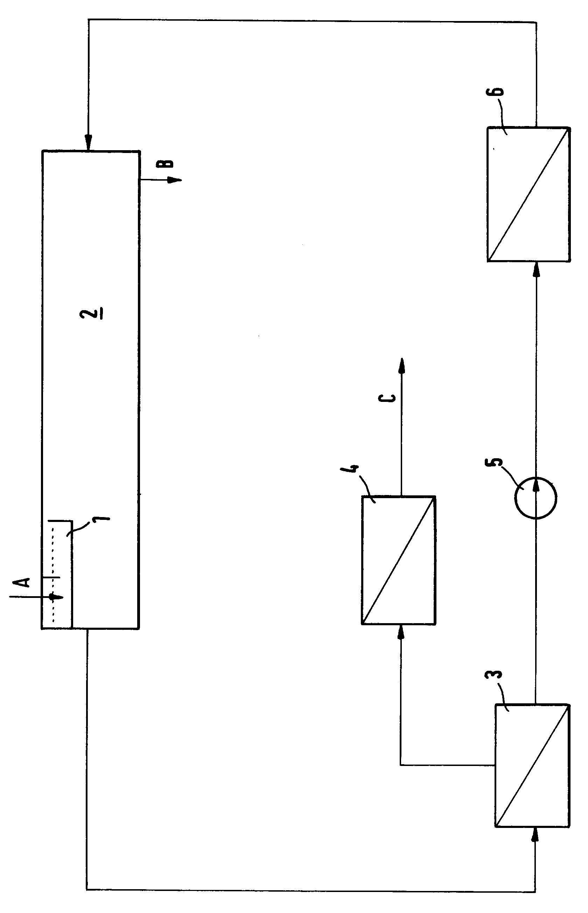

Im folgenden wird die Erfindung anhand eines in der Zeichnung schematisch dargestellten Ausführungsbeispiels näher erläutert, wobei Bezug genommen wird auf die Behandlung von Isolierschäumen aus Altkühlgeräten.

Der zu behandelnde Kunststoff-Schaum wird über eine Schleuse 1 einer Behandlungskammer 2 zugeführt und darin durch Zerkleinern aufgeschlossen, d.h. die Poren im Schaum werden geöffnet. Die Zuführung des Kunststoff-Schaums ist durch den Pfeil A angedeutet.

Die Behandlungskammer 2, die ein gegen die Umgebung abgedichtetes System bildet, ist an einen Dampferzeuger 6 angeschlossen. Der Aufschluß des Kunststoff-Schaums erfolgt in Wasserdampfatmosphäre, also bei Temperaturen oberhalb 100°. Der freiwerdende FCKW wird vom Wasserdampf aufgenommen. Die Behandlungskammer 2 weist eine zweite Schleuse (nicht dargestellt) auf, die zum Ausschleusen des behandelten Kunststoff-Schaums dient. Der Austrag des Kunststoff-Schaums ist durch den Pfeil B angedeutet. Bei dem auszuschleusenden, behandelten Kunststoff-Material handelt es sich um ein flockiges, unterhalb der Porenaufschlußgröße zerkleinertes Material. Durch ein im Austrag angeordnetes Förderorgan kann das Material verdichtet und damit von Trägermediumresten weitgehend befreit werden, wodurch sich eine sehr geringe Restfeuchte ergibt.

Das Innere der Behandlungskammer 2 kann unter Atmosphärendruck oder unter einem geringen Überdruck von ca. 0,1 bar stehen, um das Eindringen von Außenluft in das geschlossene System zu verhindern.

Die Betriebstemperatur in der Behandlungskammer 2 liegt zwischen der Sattdampftemperatur bei 1 bar und der Zersetzungstemperatur des Kunststoff-Schaums.

Das aus der Behandlungskammer 2 abgezogene Wassredampf/FCKW-Gemisch wird einem ersten Kondensator 3 zugeführt, der als Kühler-Abscheider-Kombination ausgebildet ist. In dem ersten Kondensator 3 wird das Gemisch auf eine Temperatur von 50-80°C gekühlt. Dadurch wird der kondensierende Wasserdampf von dem FCKW-Gasstrom getrennt.

Der gasförmige FCKW wird nun bei einer Temperatur von ca. 50-80°C aus dem ersten Kondensator 3 abgezogen und in einer ein- oder mehrstufigen Kondensation verflüssigt. Schematisch ist diese Kondensation durch den zweiten Kondensator 4 dargestellt, in welchem der FCKW-Gasstrom bei Temperaturen, die von den Siedepunkten zu behandelnden FCKW abhängig sind, auskondensiert.

Der zweite Kondensator 4 besteht aus Verdichter, Kühler und Abscheider. Die Leistung des Verdichters beträgt z.B. saugseitig ca. 100 Liter Gas pro Kühlgerät.

Es ist besonders vorteilhaft, daß nur der reine FCKW-Gasstrom bei diesen Temperaturen behandelt werden muß, nicht jedoch der Trägermediumstrom.

Die so gewonnene FCKW-Flüssigkeit kann dann abgefüllt und wiederverwendet werden, was durch den Pfeil C angedeutet ist.

Das im ersten Kondensator 3 gewonnene Wasser wird über eine Pumpe 5 dem Dampferzeuger 6 zugeführt und gelangt von diesem in Form von Wasserdampf in die Behandlungskammer 2. Es liegt also ein geschlossener Kreislauf vor, so daß Wasserverluste weitgehend ausgeschaltet sind.

Das erfindungsgemäße Verfahren kann besonders vorteilhaft im Rahmen einer Anlage zur umweltschonenden Aufbereitung von alten Kühlschränken eingesetzt werden.

Vorstehend ist das erfindungsgemäße Verfahren beschrieben worden mit dem Ziel, FCKW aus Kunststoff-Schäumen rückzugewinnen. Es besteht aber auch die Möglichkeit, das Verfahren immer dann anzuwenden, wenn leichtflüchtige, wasserunlösliche Stoffe von Feststoffen abzutrennen sind.

## Patentansprüche

1. Verfahren zum Rückgewinnen von halogeniertem Kohlenwasserstoff (FCKW) aus Kunststoff-Schäumen, wobei der aus dem Kunststoff-Schaum in einem gegen die Umgebung abgedichteten Prozeßraum freigesetzte FCKW in ein gasförmiges Trägermedium überführt und dann von diesem Trägermedium getrennt wird,
dadurch **gekennzeichnet,**
daß als Trägermedium Wasserdampf verwendet wird.

2. Verfahren nach Anspruch 1, dadurch gekennzeichnet, daß vor dem Überführen des FCKW in den Wasserdampf der Kunststoff-Schaum mechanisch behandelt, insbesondere zerkleinert und/oder verpreßt wird.

3. Verfahren nach Anspruch 1 oder 2, dadurch gekennzeichnet, daß die Abreicherung des FCKW-Gehalts durch den Wasserdampf unter Atmosphärendruck geschieht.

4. Verfahren nach Anspruch 1 oder 2, dadurch gekennzeichnet, daß die Abreicherung des FCKW-Gehalts durch den Wasserdampf unter einem geringen Überdruck geschieht.

5. Verfahren nach einem der Ansprüche 1 bis 4, dadurch gekennzeichnet, daß das Gemisch aus Wasserdampf und FCKW in einem Kondensator (3), der als Kühler-Abscheider-Kombination ausgebildet ist, zur Kondensation von Wasser abgekühlt wird.

6. Verfahren nach Anspruch 5, dadurch gekennzeichnet, daß der in dem Kondensator (3) gewonnene gasförmige FCKW in wenigstens einer Kondensationsstufe, die aus Verdichter, Kühler und Abscheider besteht, verflüssigt wird.

7. Verfahren nach einem der Ansprüche 5 oder 6, dadurch gekennzeichnet, daß das im Kondensator (3) gewonnene Wasser innerhalb eines Kreisprozesses über eine Pumpe (5) dem Dampferzeuger (6) und von diesem der Kunststoffschaum-Behandlung zugeführt wird.

## Claims

1. Process for the recovery of fluorochlorinated hydrocarbons (CFC) from plastic foam, wherein the hydrocarbons released from said plastic foam in a reaction vessel sealed against surrounding area are transferred into a gaseous carrier medium and then separated from this medium,
**characterized** by utilizing water vapour as carrier medium.

2. The process of claim 1, characterized in that the plastic foam is treated mechanically, in particular comminuted and/or compressed prior to the transfer of the CFC into the water vapour.

3. The process of claim 1 or 2, characterized in that the depletion of the CFC content by means of the water vapour takes place at atmospheric pressure.

4. The process of claim 1 or 2, characterized in that the depletion of the CFC content by means of the water vapour takes place at slight excess pressure.

5. The process of any of claims 1 to 4, characterized in that the mixture of water vapour and CFC is cooled in a condenser (3) for water condensation, the condenser being designed as condenser-separator combination.

6. The process of claim 5, characterized in that the gaseous CFC recovered in the condenser (3) is liquefied in at least one condensation step comprising a compressor, condenser and separator.

7. The process of claim 5 or 6, characterized in that the water recovered in the condenser (3) is transferred by a pump (5), within a cyclic process, to the vapour generator (6) and from there to the treatment of the plastic foam.

## Revendications

1. Procédé pour la récupération d'hydrocarbure halogéné (HCF) des matières plastiques mousses, dans lequel le HCF dégagé de la matière plastique mousse dans un réacteur étanche à l'environnement est transformé en un milieu porteur gazeux et ensuite séparé de celui-ci,
caractérisé en ce qu'on utilise de la vapeur d'eau en tant que milieu porteur.

2. Procédé selon la revendication 1, caractérisé en ce que, avant la transformation du HCF en vapeur d'eau, la matière plastique mousse est traitée mécaniquement, en particulier concassée et/ou comprimée.

3. Procédé selon la revendication 1 ou 2, caractérisé en ce que l'appauvrissement de la teneur du HCF par la vapeur d'eau est réalisé sous une pression atmosphérique.

4. Procédé selon la revendication 1 ou 2, caractérisé en ce que l'appauvrissement de la teneur du HCF par la vapeur d'eau est réalisé sous une légère surpression.

5. Procédé selon l'une des revendications 1 à 4, caractérisé en ce que le mélange de vapeur d'eau et de HCF est refroidi dans un condenseur (3) présentant une combinaison condenseur-séparateur, pour la condensation de l'eau.

6. Procédé selon la revendication 5, caractérisé en ce que le HCF gazeux obtenu dans le condenseur (3) est liquefié dans au moins un étage de condensation comprenant compresseur, condenseur et séparateur.

7. Procédé selon l'une des revendications 5 ou 6, caractérisé en ce que l'eau obtenue dans le condenseur (3) est amenée par une pompe (5), dans un procédé circulaire, au générateur de vapeur (6), et de celui-ci au traitement de la matière plastique mousse.
